Europäisches Patentamt

European Patent Office

Office européen des brevets

(19)

(11) Numéro de publication: **0 249 557**
A1

# DEMANDE DE BREVET EUROPEEN

(12)

(21) Numéro de dépôt: 87401314.7

(22) Date de dépôt: 11.06.87

(51) Int. Cl.⁴: **C 12 N 5/00**
C 12 P 21/00, A 61 K 39/395

(30) Priorité: 12.06.86 FR 8608494

(43) Date de publication de la demande:
16.12.87 Bulletin 87/51

(84) Etats contractants désignés:
AT BE CH DE ES FR GB GR IT LI LU NL SE

(71) Demandeur: **FONDATION CENTRE NATIONAL DE TRANSFUSION SANGUINE**
**6, rue Alexandre Cabanel**
**F-75739 Paris Cédex 15 (FR)**

(72) Inventeur: **Drouet, Xavier**
**32 Avenue Foch**
**F-94120 Fontenay (FR)**

**Goossens, Dominique**
**3 Bld de Picpus**
**F-75012 Paris (FR)**

**Rouger, Philippe**
**18 rue l'Etang Saint-Denis**
**F-92370 Chaville (FR)**

(74) Mandataire: **Ahner, Francis et al**
**CABINET REGIMBEAU 26, avenue Kléber**
**F-75116 Paris (FR)**

(54) Milieu de culture comportant de l'albumine humaine, procédé de préparation d'un produit injectable à partir de ce milieu, produit obtenu et son utilisation, composition obtenue.

(57) La présente invention concerne un milieu de culture de cellules lymphoblastoides permettant notamment de préparer des produits injectables par voie I.V. à l'homme, caractérisé en ce qu'il comporte, en plus des éléments constitutifs du milieu d'ISCOVE, à titre de protéine, uniquement de l'albumine humaine.

EP 0 249 557 A1

Bundesdruckerei Berlin

**Description**

**MILIEU DE CULTURE COMPORTANT DE L'ALBUMINE HUMAINE, PROCEDE DE PREPARATION D'UN PRODUIT INJECTABLE A PARTIR DE CE MILIEU, PRODUIT OBTENU ET SON UTILISATION, COMPOSITION OBTENUE.**

La présente invention concerne un milieu de culture de cellules lymphoblastoïdes permettant notamment d'obtenir un produit injectable par voie veineuse à l'homme.

La présente invention a été plus particulièrement réalisée dans le cadre de recherches concernant les incompatibilités de sang foeto-maternelles liées au facteur Rhésus.

Les groupes sanguins sont des systèmes antigéniques contenus à la surface des hématies; parmi ceux-ci, figure le groupe Rhésus essentiellement basé sur l'antigène Rh (D) connu depuis 1939 sous le nom de facteur Rhésus. Les personnes qui possèdent cet antigène sont dites Rhésus positif (Rh ⊕), et représentent 85 % de la population caucasienne, les autres sont Rhésus négatif (Rh ⊖).

L'importance du facteur Rh (D) tient à deux motifs essentiels :

1 - L'antigène D possède de fortes propriétés antigéniques.

2 - Le gène codant pour l'antigène D se transmet selon les lois de la génétique et se comporte comme un facteur dominant.

En particulier, l'enfant qui résulte d'une femme Rh ⊖ (15 % des femmes) avec un homme Rh ⊕ (85 % des hommes) est Rh ⊕ dans 75 % des cas.

Dans de très nombreux cas donc, la mère Rh ⊖ peut porter un enfant Rh ⊕. Cet antigène Rh ⊕ peut pour des raisons diverses et notamment lors de l'accouchement, entrer dans la circulation de la mère qui développera une allo-immunisation.

En cas de nouvelle grossesse, les anticorps ainsi produits pourront entrer dans la circulation du foetus et réagir avec les cellules du foetus pour conduire à des hémolyses massives.

Dans le cas de grossesse avec risque d'incompatibilité foeto-maternelle, on injecte actuellement des anticorps anti-Rh (D). Cette injection permet de neutraliser les éventuels globules rouges porteurs d'antigènes passant du foetus à la mère et donc de limiter les riques d'immunisation de la mère.

Jusqu'à présent, ces anticorps anti-Rhésus étaient obtenus à partir de dons de sujets Rh ⊖ ayant eu un foetus Rh ⊕ ou de sujets ayant été mis en contact avec les antigènes en cause par une autre manière. On conçoit que de telles sources d'anticorps ne soient pas satisfaisantes pour de très nombreuses raisons. C'est pourquoi on a eu recours à la technique des anticorps monoclonaux,qui permet de préparer ce type d'anticorps à partir de culture de cellules.

Les techniques de préparation des clones capables de secréter des anticorps monoclonaux humains sont maintenant assez bien connues et il n'est pas nécessaire de les décrire en détail.

L'un des problèmes important rencontré lors de la préparation de ces anticorps monoclonaux est le fait que les cultures de cellules impliquent la mise en oeuvre de protéines dans les milieux de culture dont il est difficile de se débarrasser par la suite. Ceci n'est qu'un inconvénient mineur dans les trousses à visée diagnostique, par contre cela peut devenir un inconvénient majeur pour les anticorps thérapeutiques notamment lorsqu'ils doivent être injectés.

En effet, la présence de protéines hétérologues peut en cas d'injection, notamment intraveineuse, provoquer des troubles graves qui limitent les possibilités d'utilisation des produits correspondants.

Or, dans le cas des incompatibilités foetomaternelles,il est précisément très souhaitable d'avoir recours à des injections intraveineuses.

L'un des buts de la présente invention est précisément de permettre la préparation d'anticorps monoclonaux qui peuvent être injectés à l'être humain.

Plus particulièrement, la présente invention concerne un nouveau milieu de culture permettant la production d'un produit tel que des anticorps monoclonaux humains à partir de surnageants de culture de clones de cellules lymphoblastoïdes.

Dans ce qui suit la description portera essentiellement ,à titre d'exemple ,sur l'obtention d'anticorps monoclonaux à visée thérapeutique mais,bien entendu ,ce milieu peut être utilisé pour l'obtention d'anticorps monoclonaux ayant d'autres visées ,par exemple, le diagnostic.

La composition originale du milieu selon la présente invention permet de préparer ledit produit de telle sorte qu'il soit injectable à l'homme, c'est-à-dire qu'il soit dépourvu de protéines animales et de produits toxiques. Pour ce faire, le milieu de culture selon la présente invention comporte, en plus des éléments constitutifs d'un milieu de culture de cellules lymphoblastoïdes en particulier du milieu d'Iscove, à titre de protéines uniquement de l'albumine humaine.

Le milieu d'Iscove utile à la réalisation de la présente invention est connu de l'homme de l'art. Il s'agit du milieu "Iscove Modified Dulbecco's Medium" (Référence Iscove N.N. and Melchers F, complete replacement of serum by albumin transferrin and soybean lipid in cultures of lipopolysaccharide-reactive B lymphocytes J. Exp. Med 147, 928-933, 1978) et dont la composition sera donnée en détail dans l'exemple 1.

A ce milieu, on ajoute de préférence selon la présente invention de l'albumine humaine plasmatique (fabriquée par exemple par le Centre National de Transfusion Sanguine) correspondant à la fraction V de Cohn et répondant aux caractéristiques suivantes :

. poudre

. humidité $\overline{m}$ 2 : 3 %
. pureté électrophorétique > 97,5 %
. taux d'immunoglogulines G résiduelles : < 1 %.
. taux d'alcool < 1,5 g/l pour une solution à 10 %
. dépourvue de marqueur de l'antigène Hbs et du virus LAV/HTLV III.

Cette albumine peut, d'emblée, être en solution. On l'utilise à une concentration de l'ordre de 10 à 9000mg/l, par exemple 100-700 mg/l,de préférence de 350 mg/l.

Le milieu selon la présente invention peut comporter en outre les éléments suivants :
. Au moins un acide gras insaturé.

L'acide linoléique est utilisable à ce titre à une concentration de l'ordre de 0,09 à 14 mg/l, de préférence de 2,8 mg/l. On peut substituer, aux mêmes doses, à l'acide linoléique, l'acide oléique ainsi que l'acide palmitoléique et l'acide arachidonique.

. Au moins un mercaptan, à une dose entre 0,1 et 150 µM. On peut utiliser le 2-mercaptoéthanol ou le glutathion. De préférence, selon la présente invention, on utilise du monothioglycérol à une dose de 75 µM.

. Au moins une monoamine, choisie parmi l'éthanolamine, à une dose comprise entre 300 nM et 100 µM , de préférence égale à 20 µM et la phosphoéthanolamine à une concentration comprise entre 5 et 1000 µM.

. Au moins une diamine.

Ce peut être le 1-4 diaminobutane ou le diaminopentane à une dose comprise entre 100 nM et 100 µM de préférence égale à 10 µM.

Ce peut être aussi la spermine ou la spermidine à une concentration comprise entre 10 nM et 100 µM.

Le milieu préféré selon la présente invention, appelé ci-après milieu SA, est un milieu d'Iscove auquel on a ajouté 350 mg/l d'albumine humaine plasmatique (fraction V de Cohn-procédé CNTS), 2,8 mg/l d'acide linoléique, 75 µM de monothioglycérol, 20 µM d'éthanolamine et 10 µM de 1-4 diaminobutane.

Ce milieu peut en outre être complété par addition d'un ou plusieurs ribonucléotides par exemple à une concentration de 1 à 100 mg/l.

Ce milieu permet la production d'anticorps monoclonaux humains à partir de surnageants de culture de cellules lymphoblastoïdes, cette composition permettant de préparer un produit injectable à l'homme dépourvu d'effets secondaires.

On a réalisé des études comparatives pour la culture du clone cellulaire H2D5D2 entre le milieu SA selon la présente invention et le milieu conventionnel.

Le milieu conventionnel est un milieu d'Iscove auquel on additionne 20 % de sérum de veau foetal.

Le clone H2D5D2 sécrète un anticorps monoclonal dirigé contre la facteur Rhésus (D).

Jusqu'à l'heure actuelle, il était impossible de cultiver ce clone sans ajouter au milieu d'Iscove du sérum de veau foetal dans une proportion de 20 %.

Les études comparatives montrent que le milieau SA selon la présente invention conduit à une culture du clone cellulaire H2D5D2 pratiquement identique à celle permise par le milieu conventionnel, tant du point de vue qualitatif que quantitatif.

L'un des buts de ce milieu de culture SA selon la présente invention étant de permettre la purification d'un produit tel qu'un anticorps monoclonal, et en particulier H2D5D2, afin d'obtenir un produit injectable par voie veineuse à l'homme, la présente invention concerne également le procédé de préparation et de mise en forme dudit produit.

Ainsi, après avoir obtenu le produit désiré, tel que l'anticorps monoclonal, dans un surnageant de culture réalisée en milieu SA, on procède aux étapes de purification et de mise en forme suivantes dudit produit :

- Une étape de filtration-concentration au cours de laquelle on peut procéder à une ou plusieurs filtrations puis à une concentration du produit permettant de faire varier celui-ci d'un facteur d'environ 10 à un facteur d'environ 50. Pour ce qui concerne les filtrations, on peut les réaliser sur deux membranes de 0,2 U et 0,1 U successivement.

- Une étape de purification, au cours de laquelle on soumet le produit à au moins une chromatographie sur protéine A Sépharose puis éventuellement sur colonne échangeuse d'ions.

- Une étape de mise en forme, au cours de laquelle on réalise une filtration sur membrane 0,2 U, puis on effectue une dilution pour ajuster les concentrations. A la suite de cette dilution, on ajoute de l'albumine à une concentration pouvant varier d'environ 4 à 10 g/litre. Enfin, on peut procéder à une répartition de produit pour le lyophiliser.

La présente invention concerne aussi le produit obtenu par le procédé sus-mentionné. Il s'agit de préférence de l'anticorps monoclonal en particulier anti-D tel que H2D5D2. Il peut s'agir d'un mélange de plusieurs clones ayant une même spécificité anticorps. Il est également possible d'utiliser ce type de milieu pour la culture d'autres cellules par exemple celle mentionnée dans le brevet WO-85/02413.

Enfin, la présente invention concerne l'utilisation de ce produit pour la préparation de compositions pharmaceutiques notamment injectables par voie intraveineuse, ainsi que les compositions obtenues. Ces compositions pharmaceutiques contiennent, outre le produit à titre de principe actif, les excipients pharmaceutiquement acceptables permettant leur mise en forme galénique. En particulier, on peut utiliser une solution de principe actif à base de chlorure de sodium physiologique.

La présente invention sera mieux comprise à la lecture de l'exemple suivant.

EXEMPLE :

Les essais ci-après sont des essais comparatifs entre le milieu SA selon l'invention et le milieu conventionnel à savoir : milieu d'Iscove + sérum de veau foetal (20 %).

## MODIFICATIONS DE ISCOVE DU MILIEU
## DE DULBECO

| ACIDES AMINES | mg/l | VITAMINES | mg/l |
|---|---|---|---|
| Acide L-Aspartique | 30,00 | Acide Folique | 4,00 |
| Acide L-Glutamique | 75,00 | Biotine | 0,013 |
| L-Alanine | 25,00 | Choline chlorure | 4,00 |
| L-Arginine, HCl | 84,00 | I.inositol | 7,20 |
| L-Asparagine, $H_2O$ | 28,40 | Nicotinamide | 4,00 |
| L-Cystine disodium | 82,80 | D.Calcium Pantothénate | 4,00 |
| L-Glutamine | 584,00 | Pyridoxal, HCl | 4,00 |
| Glycocolle | 30,00 | Riboflavine | 0,40 |
| L-Histidine, $HCl.H_2O$ | 42,00 | Thiamine, HCl | 4,00 |
| L-Isoleucine | 105,00 | Vitamine B12 | 0,013 |
| L-Leucine | 105,00 | | |
| L-Lysine, HCl | 146,00 | SELS ET AUTRES COMPOSANTS | |
| L-Methionine | 30,00 | | mg/l |
| L-Phenylalanine | 66,00 | $CaCl_2$ | 165 |
| L-Proline | 40,00 | KCl | 330,00 |
| L-Serine | 42,00 | $KNO_3$ | 0,076 |
| L-Threonine | 95,00 | $MgSO_4 7H_2O$ | 200,00 |
| L-Tryptophan | 16,00 | NaCl | 4505 |
| L-Tyrosine | 83,80 | $NaHCO_3$ | 3024 |
| L-valine | 94,00 | $NaH_2PO_4 2H_2O$ | 162,5 |
| | | D. Glucose | 4500,00 |
| | | HEPES | 5958,00 |
| | | Pyruvate Sodium | 110,00 |
| | | Sélènite Sodium | 0,0173 |
| | | Rouge de Phénol | 15,00 |

Il a été procédé à 10 passages du clone cellulaire H2D5D2 en partant pour chaque repiquage de la concentration de 0 5 X $10^6$ cellules/ml. Quel que soit le milieu l'inoculum est strictement identique et préparé en l'absence de sérum de veau foetal.

Ces résultats sont rapportés dans le tableau no 1 et 2.

Tableau 1 :
Détermination de la concentration cellulaire et de la viabilité au cours de 10 passages successifs.

Tableau 2 :
Détermination du taux (Ug/ml) d'immunoglobulines anti-D dans les surnageants (J7) de culture obtenue en Roller 500 ml (R500). La méthode utilisée est celle du CNRGS, conformément aux recommandations de la pharmacopée.

## TABLEAU 1

| | | Milieu d'Iscove + sérum de veau foetal 20 % | | | Milieu SA | | Technique |
|---|---|---|---|---|---|---|---|
| | | Concentration en cellules | | Viabilité | Concentration en cellules | Viabilité | |
| I | $J_3$ | | $2.27 \ 10^6$ | 94 % | $1.37 \ 10^6$ | 83 % | Flask 100 |
| II | $J_3$ | | $2.35 \ 10^6$ | 94 % | $1.24 \ 10^6$ | 76 % | Flask 150 |
| III | $J_4$ | RI | $3 \quad 10^6$ | 93 % | $1.53 \ 10^6$ | 70 % | Roller 500 |
| | $J_4$ | FIII | $1.7 \ 10^6$ | 88 % | $1.13 \ 10^6$ | 76 % | Flask 120 |
| IV | $J_4$ | RII | $2.5 \ 10^6$ | 95 % | $1.62 \ 10^6$ | 82 % | Roller 500 |
| | $J_4$ | FIV | $1.36 \ 10^6$ | 94 % | $1.14 \ 10^6$ | 86 % | Flask 150 |
| V | $J_4$ | RIII | $3.85 \ 10^6$ | 93 % | $2 \quad 10^6$ | 79 % | Roller 500 |
| | | FV | $2.15 \ 10^6$ | 90 % | $1.56 \ 10^6$ | 75 % | Flask 100 |
| VI | $J_3$ | RIV | $2.53 \ 10^6$ | 95 % | $2.07 \ 10^6$ | 84 % | Roller 500 |
| | | FVI | $1.93 \ 10^6$ | 95 % | $1.51 \ 10^6$ | 80 % | Flask 100 |
| VII | $J_4$ | RV | $3.45 \ 10^6$ | 94 % | $2.52 \ 10^6$ | 86 % | Roller 500 |
| | | FVII | $2.66 \ 10^6$ | 90 % | $1.92 \ 10^6$ | 80 % | Flask 100 |
| VIII | $J_3$ | RVI | $2.2 \ 10^6$ | 94 % | $1.5 \ 10^6$ | 83 % | Roller 500 |
| | | FVIII | $2.02 \ 10^6$ | 92 % | $1.21 \ 10^6$ | 80 % | Flask 100 |
| IX | $J_4$ | RVII | $4.06 \ 10^6$ | 93 % | $2.47 \ 10^6$ | 79 % | Roller 500 |
| | | FIX | $2.7 \ 10^6$ | 90 % | $1.40 \ 10^6$ | 71 % | Flask 100 |
| X | $J_4$ | RVIII | $4.15 \ 10^6$ | 95 % | $2.42 \ 10^6$ | 80 % | Roller 500 |
| | | FX | $2.7 \ 10^6$ | 89 % | $1.59 \ 10^6$ | 73 % | Flask 100 |

Légendes :
R : roller (encore dénommé bouteille roulante)
F : Flacon

Chiffre Romain : représente le rang de passage
J : nombre de jours depuis la date de la mise en culture

TABLEAU 2 :

| Passage | Milieu d'Iscove + sérum de veau foetal 20 % | Milieu SA |
|---|---|---|
| II | 11,2 | 11,2 |
| III | 16,8 | 11,2 |
| IV | 25,2 | 33,6 |
| V | 31,5 | 19,5 |
| VI | 21 | 15,7 |
| VII | 25,2 | 19,6 |
| VIII A | 20 | 30 |
| VIII B | 20 | 13,5 |
| Moyenne ± écart type | 21,4 ± 6,1 | 19,3 ± 8,4 |
| Test de Student | $p > 0,5$ | |

Ces tests permettent de noter que le milieu SA autorise des cultures dans des conditions quasi-identiques à celles des milieux traditionnels mais sans nécessiter l'utilisation de protéines animales. La purification des surnageants conduit à des produits qui peuvent être injectés à l'homme sans effet secondaire.

TABLEAU 3

| Repiquage n° | Concentration cellulaire à J3-J4 (10⁶/ml) | | Contration en IgG à J7 (µm/ml) | |
|---|---|---|---|---|
| | milieu SA | milieu SVF 20 % | milieu SA | milieu SVF 20 % |
| 1 | 2,05 | 3,01 | - | - |
| 2 | 1,25 | 2,18 | - | - |
| 3 | 1,15 | 2,79 | 44 | 4,4 |
| 4 | 1.49 | 1,92 | 71,2 | 14,3 |
| 5 | 1,26 | 2,82 | 49,4 | 18,1 |
| 6 | 1,06 | 2,13 | 43,4 | 14,4 |
| 7 | 1,0 | 2,40 | 39 | 8,1 |
| Moyenne + E.T. | 1,32+0,36 n = 7 | 2,46+0,41 n = 7 | 49,4+12,8 n = 5 | 11,9+5,5 n = 5 |

## Revendications

1. Milieu de culture de cellules lymphoblastoides permettant notamment de préparer des produits injectables par voie I.V. à l'homme, caractérisé en ce qu'il comporte, en plus des éléments constitutifs du milieu d'ISCOVE, à titre de protéine uniquement de l'albumine humaine.

2. Milieu selon la revendication 1, caractérisé en ce qu'il comporte en outre, au moins, un acide gras insaturé, un mercaptan, une monoamine et une diamine.

3. Milieu selon l'une quelconque des revendications 1 et 2, caractérisé en ce qu'il comporte l'albumine humaine plasmatique à raison de 10 à 9000 mg/l.

4. Milieu selon l'une quelconque des revendications 2 et 3, caractérisé en ce que l'acide gras insaturé est choisi parmi l'acide linoléique, l'acide oléique, l'acide palmitoléique ou l'acide arachidonique et à une dose comprise entre 0,09 et 14 mg/l.

5. Milieu selon l'une quelconque des revendications 2 à 4, caractérisé en ce que le mercaptan est choisi parmi le monothioglycérol, le 2 mercaptoéthanol ou le glutathion et à une dose comprise entre 0,1 et 150 µM.

6. Milieu selon la revendication 2, caractérisé en ce que la monoamine est choisie entre l'éthanolamine à raison de 300 nM à 100 µM et la phosphoéthanolamine à raison de 5 µM à 1000 µM.

7. Milieu selon la revendication 2, caractérisé en ce que la diamine est choisie parmi le 1-4 diaminobutane ou le diaminopentane à raison de 100 nM à 100 µM, la spermine ou la spermidine à raison de 10 nM à 100 µM.

8. Milieu selon l'une quelconque des revendications 1 à 7, caractérisé en ce qu'il comporte 350 mg/l d'albumine humaine plasmatique, 2,8 mg/l d'acide linoléique, 75 µM de monothioglycerol, 20 µM d'éthanolamine et 10 µM de 1-4 diaminobutane.

9. Milieu selon l'une des revendications 1 à 8, caractérisé en ce qu'il contient en outre un ou plusieurs ribonucléosides, à une concentration comprise entre 1 et 100 mg/l.

10. Procédé de préparation d'un produit, notamment injectable à l'homme, caractérisé en ce que, après avoir obtenu ledit produit dans un surnageant de culture de cellules lymphoblastoides réalisée dans un milieu selon l'une des revendications 1 à 9, on procède aux étapes successives suivantes :

    a) - étape de filtration-concentration,
    b) - étape de purification,
    c) - étape de mise en forme.

11. Procédé selon la revendication 10, caractérisé en ce que l'étape a) comprend deux filtrations successives sur membrane 0,2 U puis 0,1 U, puis une concentration avec un facteur 10 à 50.

12. Procédé selon l'une quelconque des revendications 10 et 11 , caractérisé en ce que l'étape b) comporte une chromatographie sur protéine A Sepharose puis, éventuellement, une chromatographie sur colonne échangeuse d'ions.

13. Procédé selon l'une quelconque des revendications 10 à 11, caractérisé en ce que l'étape c) comporte une filtration sur membrane 0,2 U suivie d'une dilution pour ajustement des concentrations puis une adjonction d'albumine à une concentration pouvant varier de 4 à 10 g/l suivie d'une répartition de lyophilisation.

14. Procédé selon l'une quelconque des revendications 11 à 13, caractérisé en ce que le produit préparé est un anticorps monoclonal ou un mélange d'anticorps monoclonaux à utilisation diagnostique ou thérapeutique.

15. Procédé selon la revendication 14, caractérisé en ce que l'anticorps monoclonal est un anticorps anti-D.

16. Produit obtenu par la mise en oeuvre du procédé selon l'une des revendications 10 à 15.

17. Utilisation du produit selon la revendication 16 pour la préparation de compositions pharmaceutiques, notamment injectables.

18. Composition injectable, caractérisée en ce qu'elle contient un produit selon la revendication 16.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int CI 4) |
|---|---|---|---|
| X | CHEMICAL ABSTRACTS, vol. 101, no. 25, 17 décembre 1984, page 399, résumé no. 226259g, Columbus, Ohio, US; J. MEDELSOHN et al.: "Culture of human lymphocytes in serum-free medium", & CELL. CULT. METHODS MOL. CELL BIOL. 1984, 4, 207-14 * Résumé * | 1-9 | C 12 N    5/00 C 12 P   21/00 A 61 K   39/395 |
| | --- | | |
| X | US-A-4 198 479   (TYTELL) * Colonnes 1-3 * | 1 | |
| Y | | 2-9 | |
| | --- | | |
| X | EP-A-0 060 565 (MAX-PLANCK-GESELLSCHAFT) * Revendication 6; page 23 * | 1 | |
| | | | DOMAINES TECHNIQUES RECHERCHES (Int. CI.4) |
| Y | | 2-9 | C 12 N C 12 P A 61 K |
| | ---          -/- | | |

Le présent rapport de recherche a été établi pour toutes les revendications

| Lieu de la recherche | Date d'achèvement de la recherche | Examinateur |
|---|---|---|
| LA HAYE | 22-07-1987 | SKELLY J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| Y | ANALYTICAL BIOCHEMISTRY, vol. 130, 1983, pages 445-453, Academic Press, Inc.; T. KAWAMOTO et al.: "Development of a serum-free medium for growth of NS-1 mouse myeloma cells and its application to the isolation of NS-1 hybridomas" <br> * Page 446, colonne 1, ligne 13 - colonne 2, ligne 4; page 448, colonne 1, ligne 4 - colonne 2, ligne 20; page 450, colonne 1, lignes 14-31; page 453, colonne 2, lignes 3-12 * <br><br> --- | 2-9 | |
| X | PHARMACIA FINE CHEMICALS, Presentations from Biotech '83 First World Conference and Exhibition on the commercial applications and implications of biotechnology, Londres, 4-6 mai 1983, pages 39-46, Pharmacia Fine Chemicals AB, Uppsala, SE; D. LOW: "Purification of monoclonal antibodies and their use in the isolation of biological products" <br> * Pages 41,42 * <br><br> --- | 10-14 | |
| X | WO-A-8 502 413  (STANFORD UNIVERSITY) <br> * En entier * <br><br> ---                -/- | 15-18 | |

DOMAINES TECHNIQUES RECHERCHES (Int. Cl.4)

Le présent rapport de recherche a été établi pour toutes les revendications

| Lieu de la recherche <br> LA HAYE | Date d'achèvement de la recherche <br> 22-07-1987 | Examinateur <br> SKELLY J.M. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82